# EUROPEAN PATENT APPLICATION

(11) **EP 2 645 273 A1**
(43) Date of publication of application: **02.10.2013**
(21) Application number: 11843577.5
(22) Date of filing: 06.05.2011
(51) Int. Cl.: G06F 17/30, G06Q 50/00

(54) **SYSTEM FOR DIFFERENTIATION AND ANALYSIS OF RECORDED CLINICAL DATA**

(30) Priority: 23.11.2010 RU 2010147474
(71) Applicant: Obschestvo s Ogranichennoy Otvetstvennostiu «Pravovoe Soprovojdenie Bisnesa», Moscow 119146 (RU)
(72) Inventor: GLOTKO, Vladimir Leonidovich, Dolgoprudniy 119146 (RU); RUDNEV, Alexandr Sergeevich, Moscow 117602 (RU); KARAULANOV, Anton Alexandrovich, Moscow 117420 (RU); MODENOV, Evgeniy Alexandrovich, Moscow 124617 (RU)
(74) Representative: Zimmermann & Partner
(86) International application number: PCT/RU2011/000312
(87) International publication number: WO 2012/070982

(57) **Abstract**

The invention relates to computation technology, and specifically to systems for differentiation and analysis of recorded clinical data. The technical result consists in increasing the effectiveness of the system and the reliability of the diagnosis results. The system for differentiation and analysis of recorded clinical data comprises a unit for inputting information relating to anamnesis with recording of a patient's date of presentation and complaints, a unit for inputting information relating to clinical investigations conducted by the treating doctor in relation to the patient, a unit for inputting information on the results of instrumental laboratory investigations indicating the date and procedures for conducting the investigations, a unit with information relating to treatment methods and procedures, a unit for displaying a diagnosis made by the treating doctor, a differential diagnosis matrix, a database of anatomical objects, a database of the position of anatomical objects, a database of clinical intensity indicators, a unit for describing the clinical characteristic of a symptom, a unit for indicating an anatomical object, a unit for indicating the position of an anatomical object, and a unit for the intensity of clinical indicators.

## Description

### TECHNICAL FIELD

The invention relates to medicine and can be used in ambulatory and hospital medical care as a system for differentiation and analysis of recorded clinical data (computerized clinical information-intelligent reference system, ACIRS) that allows the differentiation of clinical and instrumental laboratory data for making a diagnosis (for achieving provisional and final diagnoses). The clinical system in its automatic operating mode is designed to support medical decision making during the establishment of a diagnosis (during differential diagnostics) and the choice of (complex corrective treatment methods) a treatment methodology.

### DESCRIPTION OF THE PRIOR ART

Health facilities have lists of compulsory and additional investigations and procedures and methods of physical therapies for patients. There are recommendations and standards of the Russian Ministry of Health for the rational examination and drug treatment of various patients. The attending doctor chooses the methods of diagnosing and treating various pathologies based on his or her personal practical experiences and those of the department chief. This leads to various labor and other costs for health facilities, depending on the method chosen for one treatment regimen for one patient and updates to the treatment regimen owing to complications or comorbidities revealed during treatment, which are difficult to take into account when determining the actual scope of work for the personnel and the quality of health care. In addition, it is necessary to consider new technologies and advanced equipment, as well as their full-fledged use, which are also important for the improvement in the quality of medical services rendered.

Previous attempts to solve health care problems in terms of opportune informational support or the introduction of statistical programs have involved various forms of automation. Some of these attempts took the form of a telephone library of answers to medical questions. Other attempts were aimed at providing doctors with computerized help aids to be used during patient workups. These methods involved statistical procedures or algorithms based on the conditions of quick diagnosing and choosing a treatment method using computers and taking into account the programmed data and operating algorithm. However, the task was set to make a diagnosis automatically according to a set of standard input data about a patient by the machine, itself, and not by a doctor. Such developments function according to an algorithm of interviewing a patient to collect the necessary and sufficient set of data about the patient to the degree of saturation that corresponds to at least one of the medical diagnostic scenarios entered into the computer. This computer configuration, based on a set of medical diagnostic scenarios, envisages the doctor's participation only at the stage of entering data about the patient and ultimately leads to the formation of a "handy" feeling that everything can fit into one of the scenarios. This medical care system does not consider the participation of a doctor as a specialist or a chief participant in making a diagnosis. Furthermore, such a system implies that an efficient method has been developed to represent the medical knowledge of experts in their fields in the format of scenarios, which are constantly entered into the computer database. The scenarios must use dynamic structures for the quick and effective establishment of a patient's diagnosis.

An example of such a computerized system is the solution described in WO 98/02836, G06F19/00, published on January 22, 1998. This document describes a computerized diagnostic system comprising repetitive questions to elicit responses from a patient, with the responses establishing time-varying symptoms and each established symptom contributing a weight to a disease and elaborating one or more synergistic weights on the basis of established symptoms, with elaborating synergistic weights comprising the establishment of a synergistic symptom and the accumulation of weights of established symptoms and synergistic weights for a disease, wherein a selected set of established symptoms appearing in a specified sequence in time adds an additional diagnostic weight to a disease, thus determining whether the accumulated total weights for a disease reach or pass a threshold so as to establish and declare a diagnosis.

When forming a set of primary data about a patient and a set of symptoms, the computerized medical-condition diagnostic system forms a road map for disease treatment, which the doctor selects from a set of lists of diseases attributable in a greater or lesser degree to the indicators of the course of the disease connected to the list of symptoms formed on the basis of patient primary data. In this case, the doctor switches from one regime to the next regime (from one scenario to another) to choose a provisional diagnosis based on the assessment of combined symptoms.

This technical solution is accepted as the nearest one for the claimed object.

In the known system, diagnostics are carried out under the data scheme for disease diagnostics, which identifies an object of the first disease related to a set of symptom objects of the first disease, in which at least one symptom object of the first disease has the real weight of a symptom and indentifies an object of the second disease related to a set of symptom objects of the second disease, wherein at least one symptom object of the second disease corresponds to at least one symptom object of the first disease and has an alternative weight of a symptom. This symptom assessment algorithm makes a diagnosis by selecting a disease for which the same symptom in other diseases has a minimal value. The design of the known system comprises terminals that are equipped with data input/output devices for communication with the PC system and are connected via a remote access network to a controller for communication with a subsystem for primary data input about each patient. The subsystem comprises a unit for entering a patient's general data, a unit for entering anamnesis data of patient complaints, and a unit for entering data about patient clinical investigations conducted by the attending doctor, wherein the subsystem is connected to a database containing diagnosis scenarios and corresponding treatment methods and procedures that indicate the required pharmaceutical component.

A disadvantage of this system as a clinical information system or a reference system of information-intelligent support for the doctor when making a diagnosis and elaborating treatment methods is that it is built on the principle of arranging diseases, symptoms, and questions into a set of linked structures of diseases, symptoms, and questions, such as objects and lists, in order to access the structures and to generate a dialogue with a patient. Differentiation (or selection) in the known system is built on the principle of notion (clinical signs, symptoms) rejection. In addition, patient subjective complaints in the system are controlled, and their objectivity is established not by a medical specialist but by an automatically operated testing program. A patient may not even know where this or that organ is located; complaints about it can set a targeted testing search on the wrong track; and the system begins its search from the parameters set which leads to wasted time and possibly false results. Weights and the synergy of the weight value of this or that symptom established during clinical differentiation may be erroneous, leading to the assessment of a certain averaged value and not the condition attributable to the patient (weight establishment and the synergy of the weight value of this or that symptom are entered into the program beforehand as a certain constant coefficient). In this approach to the assessment of a patient's condition, the probability is high to establish a diagnosis attributable to this weight of a symptom (identified on the basis of questionnaires only because this manifestation is the strongest in the patient and represses psychologically less expressed forms of other symptom manifestations), but in reality this symptom may be indirect, resulting from the effect of another process. The biased description of the first frank symptoms that the system receives from a patient at the first questioning stage preconditions the retrieval of erroneous disease scenario data from the database and inaccuracy in establishing a diagnosis. The system is unable to control the (diagnostic) course of a disease and treatment results, which hinders the system's updating in terms of specifying a symptom weight or correcting a scenario.

### SUMMARY OF THE INVENTION

This invention is aimed at accomplishing a technical result consisting that improves system efficiency and authenticity of results by embodying the process of differentiation and analysis of recorded clinical data (indicators of the clinical condition of a patient, which are the data of complaints and examination by a specialist, as well as laboratory and instrumental data) by clinical matrices.

The above technical result is achieved by the system for differentiation and analysis of recorded clinical data comprising a unit for entering anamnesis data and recording the date of manifestation and patient complaints, a unit for entering data about patient clinical examinations conducted by the attending doctor, a unit for entering data on the results of instrumental laboratory investigations that indicate the date and methods of investigation, a unit with data about treatment procedures and methods, and a unit for displaying the diagnosis made by the attending doctor (or more logically, a unit for displaying differential diagnoses by the attending doctor); the system also comprising a differential diagnostic matrix, which includes databases of the clinical characteristics of symptoms, anatomical objects, positions of anatomical objects, and the intensity of clinical indicators, said databases being interconnected by a common bus wherein the matrix further comprises databases of units that describe the clinical characteristics of a symptom, indicate an anatomical object, indicate the position of an anatomical object and the intensity of clinical indicators, and that are connected by information channels to the unit for entering data about the patient's clinical examinations conducted by the attending doctor, wherein the unit that indicates the clinical characteristic of a symptom is connected to the database of the clinical characteristics of symptoms, the unit that indicates an anatomical object is connected to the database of anatomical objects, the unit that indicates the position of an anatomical object is connected to the database of the positions of anatomical objects, and the unit of the intensity of clinical indicators is connected to the database of the intensities of clinical indicators; additionally, the differential diagnostic matrix having a function of selecting data (by conjugate fields and definition) from each database that coincide with the data entered into the corresponding unit connected to this database and the function of specifying the amount of these data by reducing them according to the correcting indicators of the data selected from the next database connected to the previous database on the basis of the coincidence accuracy criterion, the coincidence authenticity criterion, the coincidence probability criterion, and the coincidence ambiguity or negation criterion.

The above signs are material and are interrelated with the formation of a stable totality of material signs sufficient for obtaining the claimed technical result (as differential diagnoses according to which a medical specialist makes decisions).

### BRIEF DESCRIPTION OF THE DRAWING

This invention is illustrated by a specific embodiment, which, however, is not the only possible one but graphically demonstrates the possibility of reaching the claimed technical result.

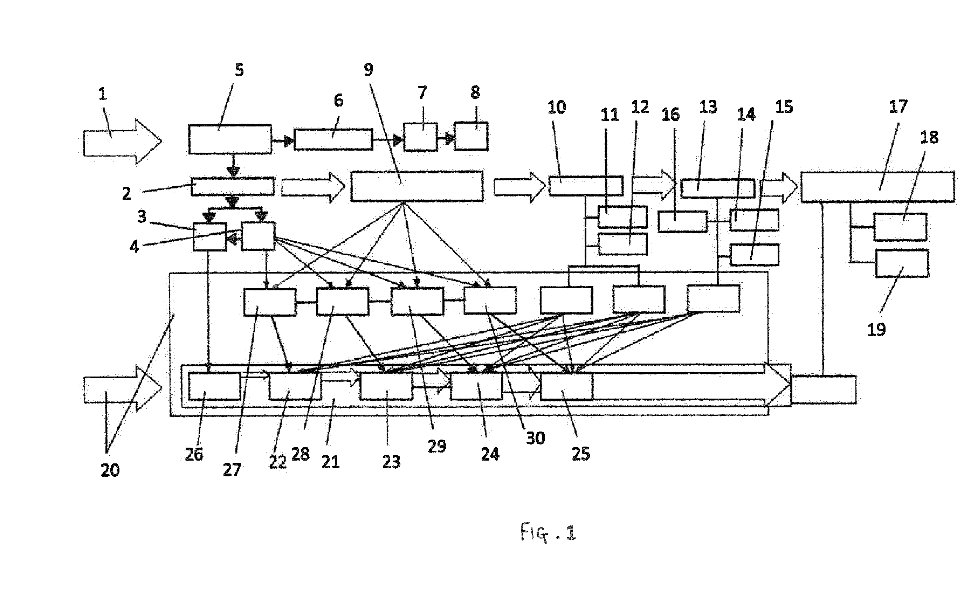

Fig. 1 shows the block diagram of a system for differentiation and analysis of recorded clinical data.

### DESCIPTION OF THE PREFERRED EMBODIMENT

According to this invention, a system for differentiation and analysis of recorded clinical data is considered, which may be used within informational (information-intelligent) support for the diagnostic and treatment process by recoding, formalizing, accumulating, exchanging, analyzing, and processing individual and group medical data in dynamics. The system is designed for everyday clinical activity of medical doctors of various specialties when conducting differential diagnostics of various diseases. The system implements the programming method of conducting differential diagnostics by groups of multiple signs and their combinations in common conjugate fields.

The basis for making scientifically grounded medical decisions in the everyday clinical practice of medical specialists is the result of conducting differential diagnostics of diseases. In most cases, an experienced doctor, after collecting anamnesis and examining a patient, usually makes a diagnosis immediately, moreover, a differentiated diagnoses. Making a differential diagnosis is a thought process that becomes practically subconscious after one acquires certain skills and experience. Due to the transience of this process, it is difficult enough to establish its regularities, but they are very important for reducing the number of erroneous cases when diagnosing, teaching differential diagnostic methods, and developing programmatic methods of comparing identified pathological changes using computer technologies.

The basis for a differential diagnosis is the recognition of symptoms on the basis of close observation of a patient's clinical picture and the accurate recording of symptoms. All of this comprises the stage of collecting and recording clinical and instrumental information on each specific patient into a database of general and specialized medical data. The next step is the analysis and evaluation of these data, which is the attempt to set the values of individual symptoms, phenomena, subjective complaints, diagnostic investigation results, provocative tests, and their interrelation.

Following data recording and evaluation are the following: differentiation; accounting the etiologic, pathogenetic, or symptomatic manifestations of known categories; and selecting the most probable among the above. It would be much easier for doctors if this process could be conducted on the basis of the laws of classical logic. Unfortunately, this is impossible. If diagnostics were able to identify phenomena and translate them into individual categories, as in mathematics and other exact sciences, there would be no need for differential diagnostics; everything would be differentiated and established easily.

For differentiation, it is necessary to know diagnostic algorithms and variants to which a given case may be attributed. Conducting differential diagnostics consists of attributing a complex of observable symptoms on the basis of available data to categories of diseases that fully or most fully correspond to the categories of diseases. Consequently, it is necessary to identify and establish a disease category and to establish a diagnosis to explain the presence of all symptoms present in a given case. To this end, diseases are categorized on the basis of certain general regularities.

An important condition in the differential analysis of medical information obtained is applying the comparative-correlative method to similar information types. Applying differential analysis to multiple signs identifies diagnostic criteria that underlie differential diagnostics.

For the convenience of differentiation using a medical database, a classifier has been developed to differentiate diseases into groups, which are subdivided into subgroups, which, in turn, are subdivided into smaller subgroups down to specific disease units. (A single method of filling in clinical and other data has been developed, that is, a matrix, which is capable of delimitatively uniting symptoms of a specific disease with all groups of other diseases in which identified symptoms are present, including the capabilities of a matrix with all disease groups, for example, heart disease, pneumonia, and peripheral nervous system disease; however, all of them are included in a single matrix). In classical logic, the basis of division is always an important or material sign. However, in practice, the most reasonable sign is chosen as the basis in most cases. In classical logic, division is done until the end on the basis of one and the same sign. In practice, when making a differential diagnosis, individual subgroups can be divided by different signs for the purposes of expedience.

When using division in practice, it is exhaustive in any case: subgroups must cover all variants encountered. We may not use a division that, for example, divides all meningitides on the basis of a cerebrospinal fluid cytogram and blood leukocytosis, because there are many variants in the course of this disease under which the results of cerebrospinal fluid investigation and blood leukocytosis practically do not differ. Division errors may be avoided using the methods of comparing, correlating, and identifying coincidences of multiple indicators and signs. These characteristic features of differential analyses of incoming medical data are programmed into the design of the claimed computerized reference system of information (information-intelligent) support for the diagnostic-treatment process.

This useful model considers a system for the differentiation and analysis of recorded clinical data that is built on the following communication and function algorithm. The system contains a unit for entering anamnesis data and recording the date of manifestation and patient complaints, a unit for entering data about patient clinical examinations conducted by the attending doctor, a unit for entering data on the results of instrumental laboratory investigations that indicate the date and methods of investigations, a unit with data about treatment procedures and methods, and a unit for displaying a diagnosis made by the attending doctor; the system also containing a differential diagnostic matrix, which includes databases of the clinical characteristics of symptoms, anatomical objects, positions of anatomical objects, and the intensity of clinical indicators; said databases being interconnected by a common bus wherein the matrix further comprises databases of units that describe the clinical characteristics of a symptom, indicate an anatomical object, indicate the position of an anatomical object and the intensity of clinical indicators, and that are connected by information channels to the unit for entering data about the patient's clinical examinations conducted by the attending doctor; wherein the unit that indicates the clinical characteristic of a symptom is connected to the database of the clinical characteristics of symptoms; the unit that indicates an anatomical object is connected to the database of anatomical objects; the unit that indicates the position of an anatomical object is connected to the database of the positions of anatomical objects; and the unit of the intensity of clinical indicators is connected to the database of the intensities of clinical indicators; additionally, the differential diagnostic matrix having a function of selecting data from each database that coincide with the data entered into the corresponding unit connected to this database and the function of specifying the amount of these data by reducing them according to the correcting indicators of the data selected from the next database connected to the previous database on the basis of the coincidence accuracy criterion, the coincidence authenticity criterion, the coincidence probability criterion, and the coincidence ambiguity or negation criterion.

Below is the example of a specific embodiment of such a system.

The system for differentiation and analysis of recorded clinical data (Fig. 1), comprising external subsystem 1 for entering patient data, which includes unit 2 for entering anamnesis data and recording the date 3 of manifestation and patient complaints 4, wherein additional data 5 about the patient's life anamnesis may be entered into this unit, indicating dates 6 of events and their nature 7, which may also be printed out on paper medium 8. The external subsystem, comprising unit 9 for entering data about patient clinical investigations conducted by the attending doctor; unit 10 for entering data on the results of instrumental laboratory investigations, indicating date 11 and methods of conducted investigations 12; unit 13 with data about treatment procedures 14 and methods 15, indicating prescription data 16; and unit 17 for displaying a diagnosis made by the attending doctor, for example, as provisional 18 and final 19 conclusions. External subsystem 2 is informationally connected to differential diagnostic matrix 20, comprising database 22 of the clinical characteristics of symptoms, database 23 of anatomical objects; database 24 with data about the positions of anatomical objects; and database 25 of the intensities of clinical indicators, said units being interconnected by common bus 21 and further connected to unit 26, indicating the complaint manifestation date obtained during the recording of the patient's anamnesis.

Differential diagnostic matrix 20 also comprises unit 27 which describes the clinical characteristic of a symptom; unit 28 which indicates an anatomical object; unit 29 which indicates the position of an anatomical object; and unit 30 which indicates the intensity of the clinical indicators; said units being connected by information channels to unit 9 for entering data about patient clinical investigations conducted by the attending doctor.

In the matrix, unit 27 which describes the clinical characteristic of a symptom is connected to database 22 of the clinical characteristics of symptoms; unit 28 which indicates an anatomical object is connected to the database 23 of anatomical objects; unit 29 which indicates the position of an anatomical object is connected to database 24 of the positions of anatomical objects; and unit 24 which indicates the intensity of a clinical indicator is connected to database 30 of the intensities of clinical indicators.

The differential diagnostic matrix has the function of selecting data from each database that coincide with data entered into the corresponding unit connected to this database and specifying the amount of these data by reducing them according to the correcting indicators of the data selected from the next database connected to the previous database on the basis of the coincidence accuracy criterion, the coincidence authenticity criterion, the coincidence probability criterion, and the coincidence ambiguity or negation criterion.

The following are selected as criteria: accurate (T) (specific, pathognomonic, obligatory); authentic (D) (of a very frequent occurrence, almost always present); probable (V) (of a frequent occurrence, sensitive); and negation (O) (contradicting, incompatible, antagonist, indicating the impossibility of the presence of this sign [symptom] under a given disease).

To compare the characteristics of several signs recorded by medical specialists in the corresponding sections of the database, the method of single conjugate fields is used. Hence, several disease units are differentiated quantitatively by the frequency of occurrence of identified signs with those available in the variants of one group of diseases.

For example, a neurologist examined a patient suspected of serous meningitis (by ICD-10, G03). The data obtained are recorded into the system by corresponding fields (in a formalized format by corresponding fields): complaints (headache features, vomiting), anamnesis (epidemic features, transmission routes, incubation period, temperature), objective status (general and neurologic examination-prevailing symptoms and syndromes, meningeal and encephalitic symptoms, their intensities (the system is based on the presence of semantics in the description of symptoms and syndromes, i.e., all clinical signs, i.e., an advice prompt of the manifestation of this or that clinical sign), the results of blood and cerebrospinal fluid laboratory investigations, the results of serologic and virologic investigations, etc. The user, i.e., the medical specialist, defines the number of conjugate fields. After the data obtained is recorded, differential diagnostics is carried out by corresponding fields (randomly selected), using the section of differential diagnostics of neurologic diseases (using the unit of differentiation of neurologic diseases by group). Medical specialists use electronic processing of data recorded into the system, correlate, and compare data collected and obtained from a patient with the available variants of the courses of the diseases of a given group (on an automatic basis) in the directory of differential diagnostics of neurologic diseases (meningitides of enteroviral etiology, parotitic etiology, lymphocytic choriomeningitis, a meningitic form of poliomyelitis, etc.). The result is transmitted by submitting provisional differential diagnoses with their probable and authentic manifestations in a given clinical case, which is automatically updated on entering additional data identified (both the symptoms of a patient's clinical condition, objectively changing in time, and the results of various investigations conducted in dynamics). Differentiation is done according to the following principle: in the unit of recording (entering) patient information, there are sections and fields identical in comparability to sections and fields in the matrix, i.e., logically conjugated. Differentiation is done according to matrix 20, the result of differential diagnostic analysis being represented, conventionally, as a graph, in which each identified disease is assessed for authenticity and probability of occurrence in a specific patient. This model makes it possible to carry out a wideband, multiple, multilevel system of differentiation, taking into account and identifying the rarest forms of the course of a disease and specific and nonspecific manifestations of pathological processes. Differentiation may also be carried out when a patient is unconscious, which is important during emergency care.

After recording all clinical signs identified in a patient as the result of examination, the system allows a clinician (i.e., rendering information-intelligent support) to assess quickly, qualitatively, and professionally the differentiation result and to identify not just several pathological processes ongoing simultaneously in one and the same patient but also the degree of intensity and manifestation in relation to one another, which predetermines the priority advantage of a corresponding specialized therapy, i.e., renders information support to a doctor during drug and other therapies at various stages of the manifestation of the same disease or during several diseases ongoing simultaneously.

Thus, the characteristic feature of the developed system is automatic search and advisory (semantic) support by entering a term during recording, differential comparison, and tracking of clinical and other data. The system ensures information-intelligent support for the diagnostic-treatment process, providing a clinician with support for presenting differentiation results as provisional diagnoses after receiving multiple clinical criteria, as follows:
- issuing general and specific symptoms and syndromes according to diagnoses presented (a preset directory, updatable on the fly);
- issuing the most authentic indicators of laboratory instrumental investigations by diagnoses submitted (a preset directory, updatable on the fly by the customer's personnel);
- generating the forms of standard patient examination under multiple differential diagnoses, highlighting the most advisable and rational investigation methods (directory elements/templates are formed by a medical facility independently, or a basic directory is formed first, and then the personnel work with it); and
- generating the forms of the primarily interrelated and most reasonable treatment of a patient under multiple differential diagnoses (directory elements/templates are formed by the clinicians of a medical facility independently, or a basic directory is formed first, and then the personnel work with it).

The subsystem components (units) are connected to one another by the algorithm of related fields, according to which a set of associated or respondent signs corresponds to each entered sign; the selection of signs is controlled by a probability scheme by which each additionally entered sign reduces the field of associated or respondent signs attributable to the first sign entered. The method of single conjugate fields ensures the possibility of differentiating several disease units quantitatively by frequency of occurrence of identified signs with those available as variants in the same group of diseases.

### INDUSTRIAL APPLICABILITY

This invention is industrially applicable in that it will help to improve the efficiency of making an authentic decision in relation to a patient and ensuring optimal treatment.

## Claims

1. The system for differentiation and analysis of recorded clinical data is **characterized by** comprising the following: a unit for entering anamnesis data and recording the date of manifestation and patient complaints; a unit for entering data about patient clinical examinations conducted by the attending doctor; a unit for entering information on the results of instrumental laboratory investigations, indicating the date and methods of investigation; a unit with data about treatment procedures and methods; a unit for displaying a diagnosis made by the attending doctor; a differential diagnostic matrix, which includes databases of the clinical characteristics of symptoms, anatomical objects, positions of anatomical objects, and the intensity of clinical indicators, said databases being interconnected by a common bus wherein the matrix further comprises databases of units that describe the clinical characteristics of a symptom, indicate an anatomical object, indicate the position of an anatomical object and the intensity of clinical indicators, and that are connected by information channels to the unit for entering data about the patient's clinical examinations conducted by the attending doctor; the matrix further comprising units that describe the clinical characteristic of a symptom, indicate an anatomical object, and indicate the position of an anatomical object and the intensity of clinical indicators, and that are connected by information channels to said unit for entering data about the patient's clinical examinations conducted by the attending doctor, wherein the unit that indicates the clinical characteristic of a symptom is connected to the database of the clinical characteristics of symptoms, the unit that indicates the position of an anatomical object is connected to the database of the positions of anatomical objects, and the unit of the intensity of clinical indicators is connected to the database of the intensities of clinical indicators; additionally, the differential diagnostic matrix having a function of selecting data from each database that coincide with the data entered into the corresponding unit connected to this database and the function of specifying the amount of these data by reducing them according to the correcting indicators of the data selected from the next database connected to the previous database on the basis of the coincidence accuracy criterion, the coincidence authenticity criterion, the coincidence probability criterion, and the coincidence ambiguity or negation criterion.
